# EUROPEAN PATENT APPLICATION

(11) **EP 3 838 334 A1**
(43) Date of publication of application: **23.06.2021**
(21) Application number: 19217281.5
(22) Date of filing: 18.12.2019
(51) Int. Cl.: A61N 1/36, A61H 3/00

(54) **SYSTEM FOR PROVIDING STIMULATION**

(71) Applicant: ONWARD Medical B.V., 5656 AE Eindhoven (NL)
(72) Inventor: CABAN, Miroslav, 1020 Renens (CH); v. ZITZEWITZ, Joachim, 1030 Bussigny (CH); DELATTRE, Vincent, 5656 AE Eindhoven (NL)
(74) Representative: DTS Patent- und Rechtsanwälte Schnekenbühl und Partner mbB

(57) **Abstract**

A system (10, 110, 210) for providing neurostimulation, comprising at least one stimulation element (12, 112, 212), the stimulation element (12, 112, 212) being configured to provide stimulation to a least one tendon and/or muscle of a patient (P).

The present invention further relates to the use of a system according to the present invention for providing stimulation and a method for providing neurostimulation.

## Description

The present invention relates to a system for providing neurostimulation, and more particularly to a system for providing stimulation to muscles and/or tendons.

The present invention further relates to the use of a system according to the present invention for providing neurostimulation.

The present invention further relates to a method for providing neurostimulation via providing stimulation at least one tendon and/or muscle.

Spinal cord injury (SCI) interrupts the communication between the spinal cord and supraspinal centers, depriving these sensorimotor circuits from the excitatory and modulatory drives necessary to produce movement.

After SCI, patients undergo intense rehabilitation in order to regain motor, autonomic and/or sensory function. Promotion of peripheral neural activity is standard practice in rehabilitation of SCI patients.

Typically, the promotion is done by at least one therapist, such as a physiotherapist, that presses against a tendon and/or a muscle of a patient with reduced control (such as motor control) to evoke a specific action (such as motor action, e.g. during a rehabilitation task). In other words, the promotion is typically done manually. However, delivering tendon and/or muscle stretches during the execution of a rehabilitation task is time-consuming and further often requires more than one therapist. It further requires a significant physical effort and good time coordination and must thus be performed by someone with enough training and skill. Frequently, at least one further therapist is required assuring patient safety and motivation.

Alternatively, the promotion may be done by epidural electrical stimulation (EES). EES is typically delivered by an active implantable medical device, such as an electrode array comprising at least one electrode, which is implanted into the epidural space. Consequently, patients must undergo an implantation procedure.

EES also stimulates peripheral neural pathways. EES as a neuromodulation strategy works - regardless of the application - by recruiting specific neuron populations through direct and indirect pathways. In the case of recovery of locomotion, EES applied over the lumbosacral spinal cord activates large-diameter, afferent fibers within the posterior roots which in turn activate motoneuron pools through synaptic connections, which in turn activate the muscles innervated by the corresponding (peripheral) neurons (Capogrosso M. et al., A Computational Model for Epidural Electrical Stimulation of Spinal Sensorimotor Circuits. Journal of Neuroscience, 33 (49) 19326-19340 (2013*).* Hence, specific spinal roots are linked to specific motor functions (Sharrard W., The segmental innervation of the lower limb muscles in man: Arris and gale lecture delivered at the royal college of surgeons of england on 2ndjanuary 1964. Ann R Coll Surg Engl, 35(2), 106-122 (1964*).*

EES increases the peripheral neural activity feeding into spinal circuits, allowing patients to perform more rehabilitation activities at a higher intensity. It is further hypothesized that the increased peripheral neural activity promotes plasticity across the lesion in the spinal cord.

The mechanism of action of EES and the tendon and/or muscle stretches are similar. Both increase the activity of peripheral neurons, namely type Ia and II sensory fibers which are part of the monosynaptic reflex pathways.

It is an object of the present invention to enable peripheral sensory fiber stimulation of a patient without requiring the manual support of one or more therapists and without the need that patients undergo surgery for implantation of an electrode array for EES.

The object is solved according to the present invention by a system with the features of claim 1. Accordingly, a system for providing neurostimulation, comprising at least one stimulation element, the stimulation element being configured to provide stimulation to a least one tendon and/or muscle of a patient.

The invention is based on the basic idea that by a stimulation element placed close to at least one tendon and/or muscle to be stimulated, stimulation may be applied from outside to the body of the patient (with a non-Invasive system) and/or automatically. In particular, this may enable time and cost-efficient optimization of stimulation, as the rehabilitation task may be performed by the patient himself without the help of another person. Further, the therapy may easily be adapted to the anatomical and/or physiological and/or pathophysiological requirements of the patient.

In particular, mechanical stimulation of the at least one tendon and/or muscle may be achieved by stretching the at least one tendon and/or muscle, for example by pushing it (forcing it to elongate) and/or by applying a vibration and/or massage and/or compression to the at least one tendon and/or muscle.

It is generally possible that tendons and/or muscles of the striated and/or smooth musculature are stimulated by the system. In other words, also inner organs may be stimulated with the system. In other words, the system may provide stimulation in order to improve motor function and/or autonomic function of a patient. Autonomic function may include sphincter function, bowel function, bladder function, ureter function, etc. The stimulation of autonomic function may be enabled by e.g. massage and/or compression of the trunk and/or parts of the trunk, such as the abdomen.

Alternatively, and/or additionally, intestinal and/or gastric glands may be stimulated by the system.

In particular, any tendon and/or muscle of a patient may be stimulated. In particular, any tendon and/or muscle of at least one arm and/or hand and/or at least one leg and/or foot of a patient may be stimulated. In particular, the at least one tendon and/or muscle may be stimulated directly and/or indirectly. In particular, stimulated muscles may include but are not limited to at least one of vastus lateralis, vastus medialis, vastus intermedius, rectus femoris, biceps femoris, semimembranosus, semitendinosus, gastrocnemius, soleus pantaris, tibialis anterior, tibialis posterior, fibularis longus, extensor digitorum longus, extensor hallucis longus, fibularis brevis, extensor hallucis longus, fibularis tertius, superior extensor retinaculum, inferior extensor retinaculum, popliteus, flexor digitorum longus, flexor digitorum brevis, flexor hallucis longus, extensor digitorum brevis, tendocalcaneus, abductor hallucis, quadratus plantae, flexor hallucis brevis, flexor digiti minimi brevis, abductor digiti minimi, deltoid, pectoralis major, biceps brachii, brachioradialis, flexor carpi radialis, flexor carpi ulnaris, extensor carpi radialis longus and/or extensor carpi radialis brevis. In particular, stimulated tendons may be any tendon related to the muscles cited above, such as the Achilles tendon and/or plantar aponeurosis.

In particular, at least one tendon and/or muscle of a leg of a patient may be stimulated to enable rehabilitation of walking, stepping, standing up, sitting down, grasping etc.

A tendons and/or muscle targeted by the system may promote a biomechanical action such as knee extension and/or flexion and/or elbow extension and/or flexion and/or ankle plantar-flexion and/or dorsi-flexion and/or hand (wrist) palmarflexion and/or dorsiflexion and/or pronation and/or supination of the forearm and/or inversion and/or eversion of the sole of the foot. In general, a biomechanical action such as abduction and/or adduction and/or elevation and/or depression and/or rotation may be promoted.

In particular, the action of a tendon may be determined by the tendon tap test.

In particular, a single tendon and/or muscle and/or a set of tendons and/or muscles, such as a pair of tendons and/or muscles associated with antagonist muscles may be stimulated by the system. In the latter case, one system may be used to promote flexion and extension of the same joint.

The system may comprise at least one fixation element to fixate the system close to the tendon and/or muscle to be stimulated. In particular, the fixation element may be or may comprise at least one of a tape, an adhesive tape and/or an adhesive element, a patch, a strap, a cuff, a belt, a clasp, a rubber band, a bandage, a garment (e.g. a sock, a stocking, tights, trousers, a shirt, a gauntlet and/or a glove) and/or any other type of fixation element. The fixation element may be at least partially made of at least one of silicone, textile, rubber, natural rubber, any type of metal and/or any kind of plastic. The fixation element may comprise elastic and/or non-elastic elements. The fixation element may comprise a closure element comprising at least one of a hook-and-loop fastener, a hook, a button, a loop and/or any other type of closure element. In particular, the fixation element may enable that the system and/or stimulation element is placed in optimal position to the tendon and/or muscle to be stimulated, without the risk of losing this optimal position.

The stimulation element may be or may comprise at least one mechanical stimulation element for stimulation of the tendon and/or muscle to be stimulated. In particular, mechanical stimulation of the tendon and/or muscle may be achieved either by directly and/or indirectly stretching a tendon and/or muscle, for example by pushing it (forcing it to elongate) and/or by applying a vibration to the tendon and/or muscle. In particular, the mechanical stimulation element may enable effects close to manual stimulation, without the requirement of therapeutic staff.

In particular, the mechanical stimulation element may be or may comprise at least one of a vibration module, a massage module, a stretching module and/or a compression module. In particular, this may enable that for each individual patient and/or each individual planned movement (including but not limited to stepping, standing up, walking, sitting down, running, swimming, grasping) and/or each individual autonomic function, the best mechanical stimulation technique (i.e. vibration, massage, stretching and/or compression) is enabled and provided by the system in order to evoke a smooth movement of the patient and/or bowel, bladder, ureter and/or sphincter function. Thus, the system comprising the mechanical stimulation element may facilitate rehabilitation and/or daily live activities.

The mechanical stimulation element may provide mechanical stimulation to the at least one tendon and/or muscle of a patient adapted to and/or synchronously with an exerted muscular activity. In particular, the system may be programmed in a temporal manner such that the correct movement is promoted by the system at the correct time during the rehabilitation and/or task execution. This may enable optimal muscular activity during rehabilitation. The rehabilitation and/or task execution may comprise any movement of daily live activities, such as walking, running, standing up, sitting down, stepping, cycling, grasping, etc.

The vibration module and/or the massage module and/or the stretching module and/or compression module may be or may comprise at least one ball and/or roll and/or block and/or clamp and/or any other element providing vibration and/or massage and/or stretching to a patient.

It is generally possible that the stimulation element is the fixation element and vice versa.

In particular at least two systems according to the present invention may be linked to each other. In particular, the linked systems may be programmed in a temporal manner such that a physiological movement based on at least two extremities (such as walking, stepping, cycling, running, swimming) is enabled.

It is possible that the system is linked to a neuromodulation system for providing EES.

In particular, the system may further comprise sensors providing feedback information.

In other words, the system may be a closed-loop system or an open-loop system.

According to the present invention, the use of a system for providing stimulation to at least one tendon and/or muscle of a patient according to any of claims 1-5 is disclosed.

In particular, the patient may be a patient with spinal cord injury (SCI).

However, it may alternatively be possible that the patient is a patient suffering from stroke, Parkinson's disease, tumors leading to partial or complete paralysis, poliomyelitis, Guillan-Barre-Syndrome, multiple sclerosis and/or cerebral palsy and/or any other disease of the central and/or peripheral nervous system causing impairment of motor function and/or autonomic function (such as bowel function and/or bladder function and/or sphincter function and/or ureter function).

According to the present invention a method is disclosed, in particular a method for providing neurostimulation, comprising at least the following steps:
- providing one stimulation element,
- using the stimulation element for stimulation to a least one tendon and/or muscle of a patient.

The method may further comprise the step of fixing the stimulation element close to the tendon and/or muscle to be stimulated.

The method may further comprise the step of providing mechanical stimulation of the tendon and/or muscle to be stimulated.

In particular, the mechanical stimulation may be or may comprise at least one of a mechanical vibration stimulation, a mechanical massage stimulation, a mechanical stretching stimulation.

In particular, the method may be performed by using the system according to one of claims 1 to 5.

Further details and advantages of the present invention shall now be disclosed in connection with the drawings.

It is shown in
- Fig. 1:: an example of promotion of peripheral neural activity of a patient according to standard practice in rehabilitation;
- Fig. 2:: an example of an embodiment of the system for providing neurostimulation according to the present invention with which the method according to the present invention can be performed;
- Fig. 3:: a further example of an embodiment of the system for providing neurostimulation according to the present invention with which the method according to the present invention can be performed;
- Fig. 4:: a further example of an embodiment of the system for providing neurostimulation according to the present invention with which the method according to the present invention can be performed; and
- Fig. 5:: an example of promotion of peripheral neural activity of a patient according to the present invention.

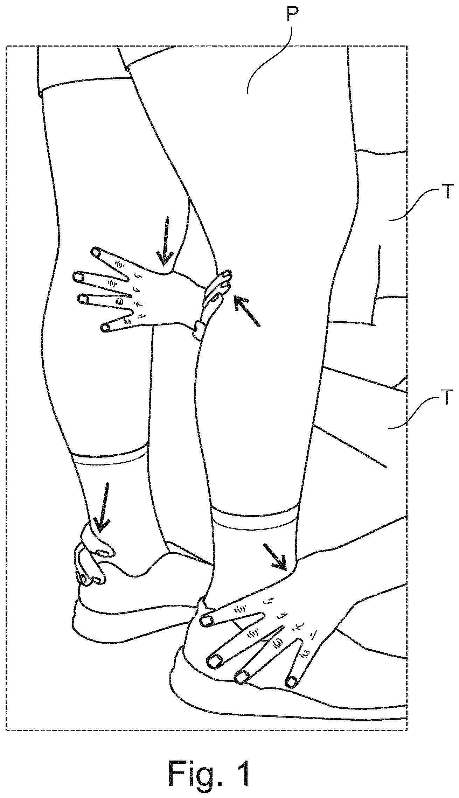

**Fig. 1** shows an example of promotion of peripheral neural activity of a patient P according to standard practice in rehabilitation.

In this embodiment, a patient P is treated manually by two therapists T.

In this embodiment, one leg of the patient P is manually treated by a first therapist T and the other leg is treated by a second therapist T.

In this embodiment, the therapists T press against tendons and/or muscles of both legs of the patient P (indicated by arrows).

Not shown in Fig. 1 is that pressing against tendons and/or muscles at least partially enables peripheral neural activity of the legs of the patient P.

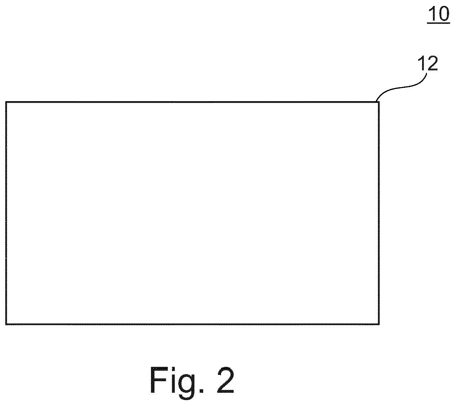

**Fig. 2** shows an example of an embodiment of the system for providing neurostimulation according to the present invention with which the method according to the present invention can be performed.

In this embodiment, the system 10 comprises a stimulation element 12.

In an alternative embodiment, the system 10 comprises more than one stimulation element 12.

It is generally possible, that the stimulation elements 12 of the system 10 are connected.

In this embodiment, the stimulation element 12 is configured to provide stimulation to at least one tendon and/or muscle of a patient P.

Not shown in Fig. 2 is that the stimulation element 12 is a mechanical stimulation element 12 for stimulation of the tendon and/or muscle to be stimulated.

Alternatively, the stimulation element 12 could comprise a mechanical stimulation element 12 for stimulation of the tendon and/or muscle to be stimulated.

Not shown in Fig. 2 is that the stimulation element 12 could generally be or comprise at least one mechanical stimulation element 12.

Not shown in Fig. 2 is that in this embodiment the mechanical stimulation element 12 is a massage module.

In an alternative embodiment, the mechanical stimulation element 12 could comprise a massage module.

In an alternative embodiment, the mechanical stimulation element 12 could be a vibration module and/or a stretching module and/or a compression module.

In general, the mechanical stimulation element 12 could be or could comprise at least one of a vibration module, a massage module, a stretching module, a compression module.

In general, the system 10 could be used for providing stimulation to a least one tendon and/or muscle of a patient P.

In this embodiment, the patient P is a patient P with spinal cord injury (SCI).

In general, the system 10 could perform a method for providing stimulation, comprising the following steps:
- Providing one stimulation element 12,
- Using the stimulation element 12 for stimulation to a least one tendon and/or muscle of a patient P.

The method could further comprise the step of providing mechanical stimulation of the tendon and/or muscle to be stimulated.

The mechanical stimulation could be or could comprises at least one of a mechanical vibration stimulation, a mechanical massage stimulation, a mechanical stretching stimulation, a mechanical compression module.

In general, the method could be performed by using the system 10, 10, 210 according to one of Figs. 2-5.

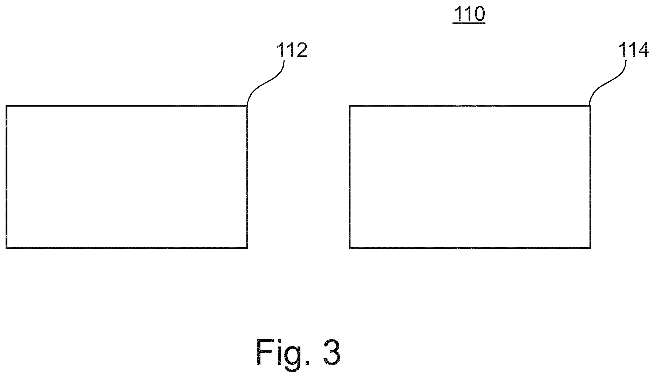

**Fig. 3** shows a further example of an embodiment of the system for providing neurostimulation according to the present invention with which the method according to the present invention can be performed.

The system 110 comprises the structural and functional features as disclosed for the system 10 disclosed in Fig. 2. The corresponding references are indicated as 100+x (e.g. stimulation element 112).

In this embodiment, the system 110 further comprises a fixation element 14.

In this embodiment, the fixation element 114 fixates the system 110 close to the tendon and/or muscle to be stimulated.

Not shown in this embodiment is that the fixation element 114 and the stimulation element 112 are connected directly.

In an alternative embodiment, the fixation element 114 and the stimulation element 112 could be connected indirectly.

Not shown in Fig. 3 is that the system 110 could generally comprise more than one fixation element 114 and/or more than one stimulation element 112.

In general, the system 110 could perform a method for providing neurostimulation, comprising the following steps:
- providing one stimulation element 112,
- using the stimulation element 112 for stimulation to a least one tendon and/or muscle of a patient P.

The method could further comprise the step of fixing the stimulation element 112 close to the tendon and/or muscle to be stimulated.

Not shown in Fig. 3 is that the system 110 is typically fixed close to the ankle and/or knee and/or wrist and/or elbow of a patient P.

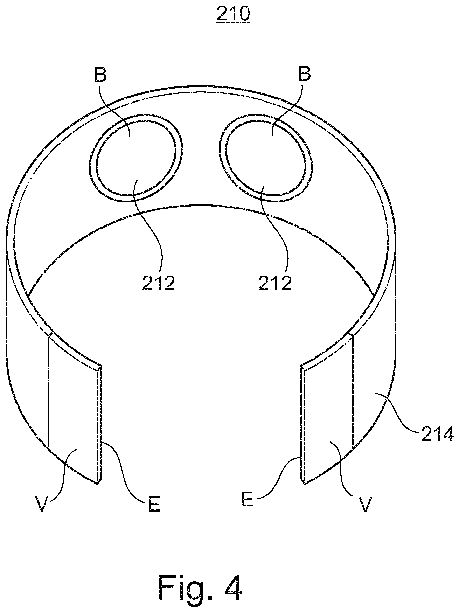

**Fig. 4** shows a further example of an embodiment of the system for providing neurostimulation
according to the present invention with which the method according to the present invention can be performed.

The system 210 comprises the structural and functional features as disclosed for the system 110 disclosed in Fig. 3. The corresponding references are indicated as 100+x (e.g. stimulation element 212).

In this embodiment, the fixation element 214 is an elongated strap 214 provided with a hook-and-loop fastener system V at its ends E to releasably connect the ends E of the strap 214 with each other.

In an alternative embodiment, the ends E of the strap 214 may be connected by any other type of lock, including but not limited to at least one hook, eyelet, button and/or knot.

In an alternative embodiment, the strap 214 may be a circular strap 214.

In general, the fixation element 214 could be a circular fixation element 214.

In general, the fixation element 214 could comprise a fastener system V.

In this embodiment, the strap 214 is at least partially made of elastic material.

In an alternative embodiment, the strap 214 is completely made of elastic material.

In an alternative embodiment, the strap 214 is made of non-elastic material.

In this embodiment, the strap 214 is configured and arranged for fitting to a patient's ankle.

In an alternative embodiment, the fixation element 214 could be configured and arranged for fitting at any part of the patient's P body.

In this embodiment, the stimulation element 212 is a mechanical stimulation element 212.

In particular, in this embodiment, the mechanical stimulation element 212, which is a massage module, comprises two balls 8, in particular two shiatsu balls.

In this embodiment, the mechanical stimulation element 212 is movably connected with the fixation element 14.

In this embodiment, the mechanical stimulation element 212 is configured and arranged to provide forces to at least one tendon and/or muscle of a patient equipped with the system 210.

In this embodiment, the mechanical stimulation element 212 is configured and arranged to provide forces to the Achilles tendon of a patient P equipped with the system 210.

In this embodiment, the mechanical stimulation element 212 is configured and arranged to squeeze and/or knead the Achilles tendon of a patient P equipped with the system 210.

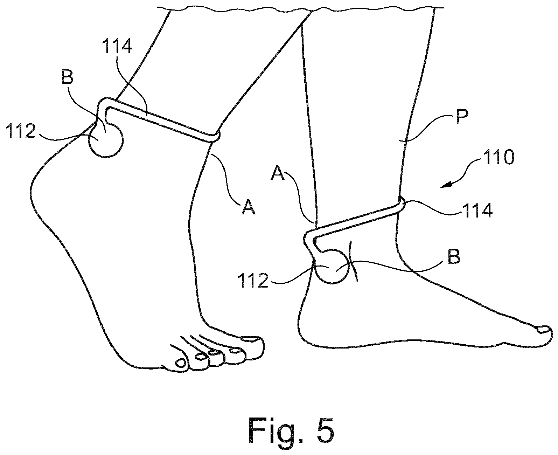

**Fig. 5** shows an example of promotion of peripheral neural activity of a patient P according to the present invention.

In this embodiment, a patient P is equipped with the system 110 disclosed in Fig. 3.

In this embodiment, the system 110 comprises two stimulation elements 112 and two fixation elements 114.

In this embodiment, each ankle A of the patient P is equipped with one stimulation element 112 and one fixation element 114.

In this embodiment each stimulation element 112 comprises a massage module.

Not shown in Fig. 5 is that the massage module comprises two massage balls B.

Not shown in Fig. 5 is that one massage ball B is located at each side of the Achilles tendon of the patient P (one on the left side of the Achilles tendon and one on the right side of the Achilles tendon).

Not shown in Fig. 5 is that alternatively and/or additionally, any other tendon and/or muscle and/or organ of the patient P could be stimulated with the system 110, given that the fixation element 114 places the system 110 close to the respective tendon and/or muscle and/or organ.

In this embodiment, the fixation element 114 is a U-shaped cuff.

In other words, in this embodiment, the fixation element 114 is not closed but placed around the ankle.

Not shown in Fig. 5 is that the fixation element 114 is a non-elastic cuff.

However, in an alternative embodiment, the fixation element 114 may be an elastic cuff.

In this embodiment, the massage balls B squeeze against the Achilles tendon.

In an alternative embodiment, the squeeze could be generated by the cuff itself.

In other words, in an alternative embodiment, the fixation element 114 could be the stimulation element 112 (and vice versa).

Not shown in Fig. 5 is that the system 110 delivers sensory fiber stimulation by mechanically stimulating the Achilles tendons of the patent P.

Not shown in Fig. 5 is that the system 110 could be programmed in a temporal manner such that a correct movement involving both legs of the patient P (e.g. stepping) is promoted at the correct time during execution of a rehabilitation task.

Further not shown in Fig. 5 is that the mechanical stimulation element could provide mechanical stimulation to the at least one tendon and/or muscle of a patient adapted to and/or synchronously with an exerted muscular activity.

### References

- 10, 110, 210: system
- 12, 112, 212: stimulation element/ mechanical stimulation element
- 14, 114, 214: fixation element

- A: ankle
- B: massage ball
- E: end of fixation element
- P: patient
- T: therapist
- V: hook-and-loop fastener

## Claims

1. A system (10, 110, 210) for providing neurostimulation, comprising at least one stimulation element (12, 112, 212), the stimulation element (12, 112, 212) being configured to provide stimulation to a least one tendon and/or muscle of a patient (P).

2. The system (10, 110, 210) according to claim 1, **characterized in that** the system (10, 110, 210) comprises at least one fixation element (14, 114, 214) to fixate the system (10, 110, 210) close to the tendon and/or muscle to be stimulated.

3. The system (10, 110, 210) according to claim 1 or claim 2, **characterized in that** the stimulation element (12, 112, 212) is or comprises at least one mechanical stimulation element (12, 112, 212) for stimulation of the tendon and/or muscle to be stimulated.

4. The system (10, 110, 210) according to claim 3, **characterized in that** the mechanical stimulation element (12, 112, 212) is or comprises at least one of a vibration module, a massage module, a stretching module, a compression module.

5. The system (10, 110, 210) according to claim 4, **characterized in that** the mechanical stimulation element (12, 112, 212) is configured to provide mechanical stimulation to the at least one tendon and/or muscle of a patient adapted to and/or synchronously with an exerted muscular activity.

6. The use of a system (10, 110, 210) according to one of the preceding claims for provide stimulation to a least one tendon and/or muscle of a patient (P).

7. The use of claim 6, **characterized in that** the patient is a patient with spinal cord injury (SCI).

8. A method for providing neurostimulation, comprising at least the following steps:
- providing one stimulation element (12, 112, 212),
- using the stimulation element (12, 112, 212) for stimulation to a least one tendon and/or muscle of a patient (P).

9. The method according to claim 8, **characterized in that** the method further comprises the step fixing the stimulation element (12, 112, 212) close to the tendon and/or muscle to be stimulated.

10. The method according to claim 8 or claim 9, **characterized in that** the method further comprises the step of providing mechanical stimulation of the tendon and/or muscle to be stimulated.

11. The method according to claim 10, **characterized in that** the mechanical stimulation is or comprises at least one of a mechanical vibration stimulation, a mechanical massage stimulation, a mechanical stretching stimulation.

12. The method according to one of claims 8 to 11, **characterized in that** the method is performed by using the system (10, 110, 210) according to one of claims 1 to 5.
